# EUROPEAN PATENT SPECIFICATION

(11) **EP 2 514 430 B1**
(45) Date of publication and mention of the grant of the patent: **10.09.2014**
(21) Application number: 10837763.1
(22) Date of filing: 16.09.2010
(51) Int. Cl.: A61K 36/185, B01D 11/00, B01D 9/00

(54) **METHOD AND APPARATUS FOR SIMULTANEOUSLY MANUFACTURING ULTRA PURE BETULIN AND HIGHLY PURE BETULINIC ACID FROM BIRCH BARK**
VERFAHREN UND VORRICHTUNG ZUR GLEICHZEITIGEN HERSTELLUNG VON ULTRAREINEM BETULIN UND HOCHREINER BETULINSÄURE AUS BIRKENRINDE
PROCÉDÉ ET APPAREIL POUR LA PRODUCTION SIMULTANÉE DE BÉTULINE ULTRA PURE ET D'ACIDE BÉTULINIQUE HAUTEMENT PUR À PARTIR D'ÉCORCE DE BOULEAU

(30) Priority: 14.12.2009 KR 20090123831
(43) Date of publication of application: 24.10.2012
(73) Proprietor: Korea Institute of Energy Research, Daejon 305-343 (KR)
(72) Inventor: CHUE, Kuck-Tack, Daejeon 305-762 (KR); PARK, Jong-Kee, Daejeon 305-769 (KR); KIM, Tae-Hwan, Daejeon 305-761 (KR); YI, Chang-Keun, Daejeon 305-720 (KR)
(74) Representative: Neugebauer, Jürgen
(86) International application number: PCT/KR2010/006329
(87) International publication number: WO 2011/074766

(56) References cited:
- KR-A- 20030 067 545
- KR-B1- 100 770 940
- KR-B1- 100 827 072
- US-B1- 6 280 778
- US-B1- 6 392 070
- US-B2- 6 634 575
- US-B2- 6 815 553
- US-B2- 7 246 184

## Description

### Technical Field

The present invention relates to a method and apparatus for simultaneously producing ultrapure betulin and highly-pure betulinic acid from birch tree bark, and more particularly, to a method and apparatus for simultaneously producing ultrapure betulin and highly-pure betulinic acid from birch tree bark, which can continuously produce ultrapure betulin and highly-pure betulinic acid by crystallizing highly-pure betulin included in a crude extract obtained from the outer and inner bark of a birch tree by a mixture of polar organic solvents and then separating polar pigment components from the crystallized highly-pure betulin using a 4-bed eluent swing continuous adsorption process.

### Background Art

A large amount of literature has been published regarding the scientific fact that betulin is generally used as a starting material used in the organic synthesis of betulinic acid, butulonic acid, or derivatives thereof, each of which is effectively used as a therapeutic agent. Further, a large number of theses have been published regarding the scientific discovery that betulinic acid has antitumor activity to melanomas (for example, MEL-2 and MEL-4) [Pisha, E. et al., (1995) J. M. Nature Medicine, 1, 1046-1051], and the research results that betulinic acid has anti-HIV activity to a Hg lymphocytic cell [Fujioka, T. et al., (1994) J. Nat. Prod. 57, 243-247]. Further, the paper "Antiviral Research 64 (2004) 127-130, Yunhao Gong et al." disclosed that antiviral effects were provided to herpes simplex viruses when betulin was mixed with acyclovir and then used. Moreover, recently, in 2006, US 2006/0154903 Al (Brij B. Saxena) disclosed that butulonic acid or a lysinated derivative has an effect on inhibiting the growth of human prostatic cancer cells (LNCaP, PC-3, and DU-145) and fibroblast cells. Further, PCT/US2005/039068 (WO2006/050158 A2), invented by GLINSKI, disclosed a technology of applying betulin ether derivatives to functional cosmetics in order to obtain the effects of a skin and hair protector, a skin moisturizer, an antiperspirant, an anti-wrinkle treatment cream, and a hair depilatory. Furthermore, inventors (Graham P. Allaway et al.) insisted in US 7,365,221 B2 that derivatives of monoacylated betulin and dihydrobetulin have potent anti-HIV activity.

As a conventional method of producing betulin or betulinic acid, US 7,246,184 B2, invented by Pavel A. Krasutsky et al., disclosed a technology of separating outer bark from a birch tree and then separating betulin (at a purity of 90% or more, and a yield of 25% based on outer bark), lupeol (at a purity of 90%, and a yield of 1.7% based on outer bark) and betulinic acid (at a purity of 95%, and a yield of 1.6% based on outer bark) from the outer bark using supercritical carbon dioxide as a solvent. Here, the yield is defined as (total product amount x purity)/(raw material amount x concentration) x 100, and is represented by percentage (%).

Further, Korean Patent Nos. 10-0827072 and 10-0770940 disclosed technologies of producing betulin by applying a mixture of hexane (nonpolar solvent) and ethyl acetate (polar solvent) and ultrasonic assisted extraction to the bark of a birch tree growing naturally in Korea. Each of the technologies includes the steps of: obtaining a crude extract from the bark of a birch tree using a pure polar solvent and reflux extraction; removing nonpolar materials from the crude extract using a mixture of hexane (nonpolar solvent) and ethyl acetate (polar solvent); and isolating betulin having a purity of 95% or less using a general open column charged with silica gel. However, these technologies are problematic in that it is difficult to continuously produce a product having purity of 99%.

Further, in conventional technologies, a crude extract is crystallized using the difference in solubility occurring when the crude extract is dissolved in a single polar solvent (for example, alcohol such as isopropanol or the like) near a boiling point and then cooled. The crystallized extract obtained in this way includes betulin having low purity of 90%. Further, a recrystallization method using a single solvent is disadvantageous compared to a recrystallization method using a solvent mixture in that solvent consumption is high, so crystallization efficiency is low, thereby increasing the production cost of betulin.

Further, the above-mentioned conventional technologies are problematic in that a large amount of a solvent is consumed because a mixed solvent of a polar solvent and a nonpolar solvent is used in order to remove nonpolar components, and in that betulin having a purity of 95% is noncontinuously produced because a single open column is charged with a fixed bed.

### Disclosure

### Technical Problem

Accordingly, the present invention has been devised to solve the above-mentioned problems, and an object of the present invention is to provide a method and apparatus for simultaneously producing ultrapure betulin and highly-pure betulinic acid from the bark of a birch tree, which can simultaneously and continuously produce betulin having purity of 99.5% or more and betulinic acid having purity of 95% or more.

Another object of the present invention is to provide a method and apparatus for simultaneously producing ultrapure betulin and highly-pure betulinic acid from the bark of a birch tree, which can crystallize betulin having purity of 95% or more by dissolving a crude extract extracted from the inner and outer bark of a birch tree in a polar mixed solvent and recystallizing the dissolved crude extract.

Still another object of the present invention is to provide a method and apparatus for simultaneously producing ultrapure betulin and highly-pure betulinic acid from the bark of a birch tree, which can produce ultrapure betulin and highly-pure betulinic acid through one column line, and which can continuously produce betulin and betulinic acid by regenerating the other column line through which ultrapure betulin and highly-pure betulinic acid are produced.

### Technical Solution

In order to accomplish the above objects, an aspect of the present invention provides a method of simultaneously producing ultrapure betulin and highly-pure betulinic acid from bark of a birch tree, including the steps of: preparing a crude extract from bark of a birch tree pulverized by a single polar organic solvent or a mixed polar organic solvent; crystallizing the crude extract by extracting the crude extract using a mixed polar organic solvent and then filtering and drying the crude extract, so as to obtain a crystallized product; dissolving the crystallized product in a single polar organic solvent or a mixed polar organic solvent to form a feed solution; and separating the feed solution using a 4-bed eluent swing continuous adsorption process to simultaneously produce ultrapure betulin having purity of 99.5% or more and highly-pure betulinic acid having purity of 95% or more.

In the method, a crystallized product including highly-pure betulin having purity of 95% or more can be obtained by dissolving and crystallizing the crude extract extracted from the bark of a birch tree (specifically, *betula papyrifera or betula platyphylla*) using the mixed polar organic solvent.

Further, in the method, the solubility of betulin in the mixed polar organic solvent is synergistically increased by extracting the crude extract using the mixed polar organic solvent, so that the consumption of a solvent can be reduced, and the crystallized product including 94 ∼ 96.5% of betulin, 3 - 4% of betulinic acid and residual polar pigment components can be produced from the bark of a birch tree.

That is, when a single polar organic solvent is used, the solubility of betulin in methanol is 6.73 g/L or less at 64.7°C, the solubility of betulin in chloroform is 40.69 g/L or less at 60.5°C, the solubility of betulin in ethanol is 18.49 g/L or less at 78°C, and the solubility of betulin in ethyl acetate is 23.02 g/L or less at 77.1°C. In contrast, when a mixed solvent of chloroform and ethanol is used as the mixed polar organic solvent of the present invention, the solubility of betulin in the mixed solvent of chloroform and ethanol (18:82 v/v, density = 0.91 g/mL) is 38.6 g/L at 60.5°C, the solubility of betulin in the mixed solvent of chloroform and ethanol (57:43 v/v, density = 1.19 g/mL) is 140.0 g/L at 60.5°C, and the solubility of betulin in the mixed solvent of chloroform and ethanol (82:18 v/v, density = 1.37 g/mL) is 162.8 g/L at 60.5°C.

Further, when ethanol is used in order to crystallize highly-pure betulin, 1.0 g of the crude extract must be dissolved in 55 mL of ethanol at 78°C. When the mixed solvents of chloroform and ethanol, respectively having densities of 0.91, 1.19 and 1.37 g/mL, are used, 26, 7.1 and 6.2 mL of mixed solvents are required, respectively. When these mixed solvents are used, the consumption of a solvent can be reduced by 47.3%, 87.1% and 88.7%, respectively.

Further, the method of the present invention is advantageous in that betulin having purity of 99.5% or more and betulinic acid having purity of 95% or more, helpful to the human body, can be simultaneously produced from the bark of a birch tree by removing a small amount of impurities, such as polar pigment components, from the product obtained in the recrystallization process.

Further, the method of the present invention is advantageous in that, when the crystallized product is dissolved in a polar solvent such as acetone or ethanol and then treated with polar active alumina or silica gel, betulin having purity of 99.5% or more and betulinic acid having purity of 95% or more can be obtained, thus improving the production yield of betulin and betulinic acid.

Further, the method of the present invention is advantageous in that ultrapure betulin and highly-pure betulinic acid can be produced by one column line, and simultaneously the other column line, by which ultrapure betulin and highly-pure betulinic acid were produced, can be regenerated, thus continuously producing ultrapure betulin and highly-pure betulinic acid.

### Description of Drawings

FIG. 1 is a block diagram showing a method of producing betulin and betulinic acid according to the present invention;
FIG. 2 is a graph showing the calibration curve of betulinic acid according to the present invention;
FIG. 3 is a graph showing the calibration curve of betulin according to the present invention;
FIG. 4 is a view showing the results of Example 2;
FIG. 5 is a view showing the results of Example 3;
FIG. 6 is a view showing the results of Example 4;
FIG. 7 is a view showing the results of Example 5;
FIG. 8 is a view showing the results of Example 6; and
FIG. 9 is a view showing the results of Example 7.

### Best Mode

The method of simultaneously producing ultrapure betulin and highly-pure betulinic acid from bark of a birch tree according to the present invention is configured such that a crude extract including betulin (CAS 473-98-3), betulinic acid (CAS 472-15-1) and polar pigment components are prepared from the bark (mainly outer bark) of a birch tree naturally growing in the northern hemisphere of 34° north latitude, such as Korea, China, Europe and the like, using a polar organic solvent, and highly-pure betulin included in this crude extract is crystallized using a mixed polar organic solvent to obtain a crystallized product, and then ultrapure betulin and highly-pure betulinic acid are simultaneously produced from the crystallized product including highly-pure betulin as a major component.

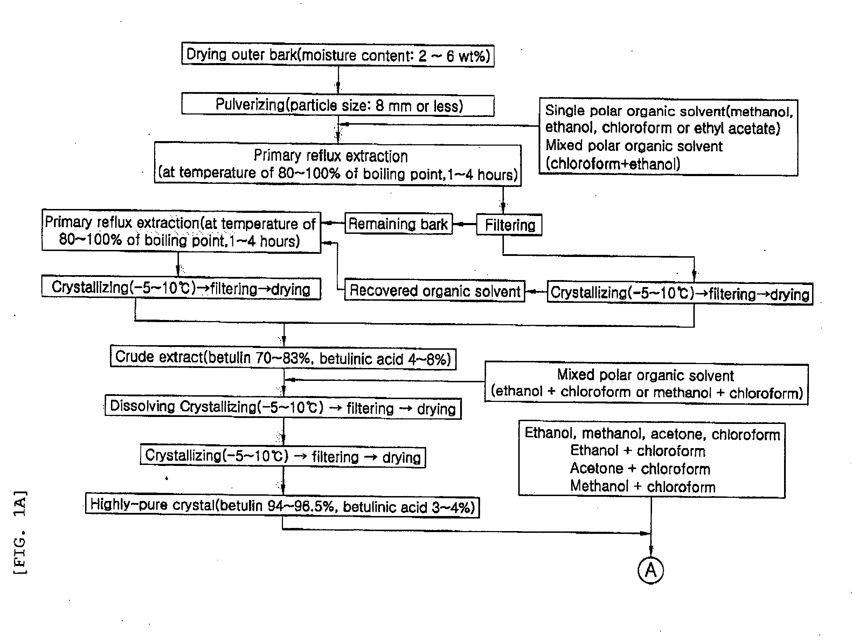

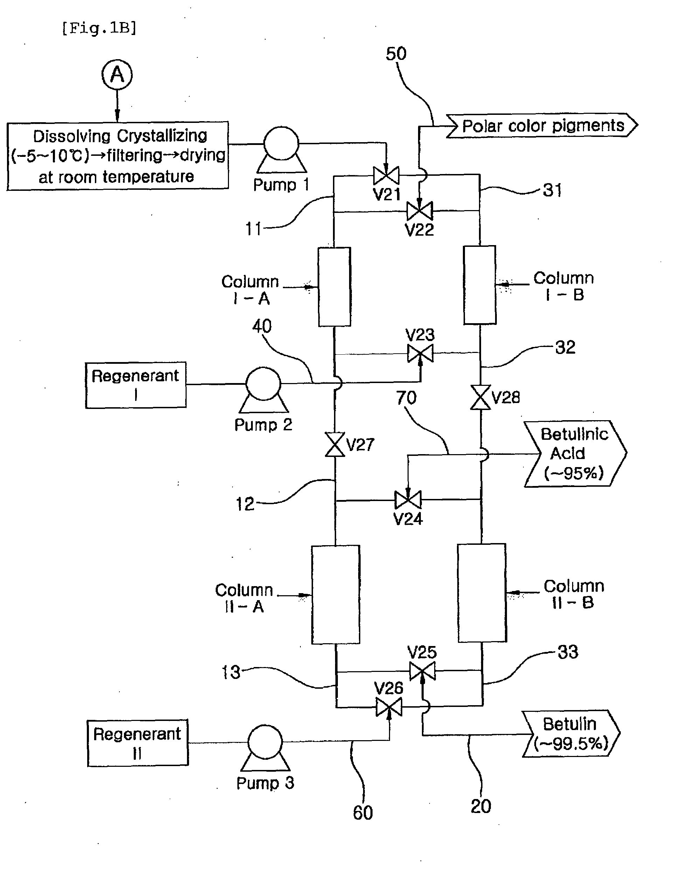

FIG. 1 is a block diagram showing a method of producing betulin and betulinic acid using a 4-bed eluent swing continuous adsorption process according to the present invention. The method is configured such that ultrapure betulin having purity of 99.5% or more and highly-pure betulinic acid having purity of 95% or more can be simultaneously produced from the pulverized bark of a birch tree. Here, yield means weight percentage (wt%), amount (for example, amount of betulin) means concentration ratio, and mixing ratio of solvents means volume ratio (v/v).

The method of producing ultrapure betulin and highly-pure betulinic acid includes the steps of: 1) preparing a crude extract from birch bark particles pulverized to a size of 8 mm or less using a reflex extractor; 2) producing a crystallized product including 94 ∼ 96.5% of betulin and 3 ∼ 4% of betulinic acid from the crude extract using a mixed polar solvent; and 3) producing ultrapure betulin and highly-pure betulinic acid from a feed solution obtained by dissolving the crystallized product in a single polar organic solvent or a mixed polar organic solvent using a 4-bed eluent swing continuous adsorption process.

More concretely, the method of producing ultrapure betulin and highly-pure betulinic includes the steps of: preparing a crude extract from bark of a birch tree pulverized by a single polar organic solvent or a mixed polar organic solvent; crystallizing the crude extract by extracting the crude extract using a mixed polar organic solvent and then filtering and drying the crude extract, so as to obtain a crystallized product; dissolving the crystallized product in a single polar organic solvent or a mixed polar organic solvent to form a raw material solution; and separating the raw material solution using a 4-bed eluent swing continuous adsorption process to simultaneously produce ultrapure betulin and highly-pure betulinic acid.

In the step of preparing the crude extract, dry birch tree outer bark having a moisture content of 2 ∼ 6% is finely pulverized and sieved to form particles having a size of 8 mm or less. The particles are charged in a reflux extractor together with a single polar organic solvent such as ethanol, methanol, chloroform, ethyl acetate or the like, or a mixed polar organic solvent thereof, and are then heated for 1 ∼ 4 hours, and typically 1 hour, at a temperature of 80 ∼ 100% of the boiling point of a polar solvent (for example, in the case of ethanol, 63 ∼ 78.4°C; in the case of methanol, 52 ∼ 64.7°C; in the case of chloroform, 49 ∼ 61.2°C; and in the case of ethyl acetate, 62 ∼ 77.1°C) to extract betulin, betulinic acid and polar pigment components into a polar organic solvent.

Further, a mixture of polar organic solvents, in which a birch tree outer bark (solid), betulin, betulinic acid and polar pigment components are dissolved, is filtered using a 0.5 µm PTFE membrane or a porous glass fritted filter. In this case, the birch outer bark soaked with the polar organic solvent filtered by hot filtering is stored in a batch-type extraction tank, and is used to conduct secondary extraction.

The filtered solution includes a polar organic solvent, betulin, betulinic acid and polar pigment components. 40 ∼ 80% of the total amount of the polar organic solvent is evaporated using a vacuum rotary evaporator. In this case, the polar organic solvent vapor discharged from the vacuum rotary evaporator is heat-exchanged with cooling water to be liquefied. The liquefied polar organic solvent vapor is used to secondarily extract the birch tree outer bark stored in the batch-type extraction tank.

Subsequently, the filtered solution, including betulin, betulinic acid and polar pigment components, which is concentrated by the vacuum rotary evaporator, is left at a temperature of -2 ∼ 10°C for 10 ∼ 24 hours to form crystals in the polar organic solvent. The crystals are filtered by a 0.5 µm PTFE membrane or a porous glass fritted filter and then dried to obtain a primary crude extract.

Meanwhile, the filtered solution remaining after obtaining the primary crude extract or the filtered solution and residual bark is charged in the reflux extractor again, and is then heated to about 80°C for 20 ∼ 40 minutes (typically, about 30 minutes) to re-extract betulin, betulinic acid and polar pigment components into the polar organic solvent.

The solution including the mixture re-extracted into the polar organic solvent is filtered, and then 40 ∼ 80% of the total amount of the polar organic solvent is evaporated from the filtered solution using a vacuum rotary evaporator to concentrate betulin, betulinic acid and polar pigment components in the filtered solution. The concentrated filtered solution is left at a temperature of -2 ∼ 10°C for 3 ∼ 5 hours (preferably, about 4 hours) to form crystals in the polar organic solvent. The crystals are filtered and dried to obtain a secondary crude extract.

Further, the filtered solution remaining after obtaining the secondary crude extract is completely evaporated. In this case, the solvent is recovered and then reused, and the solids are discarded. The recovered solvent and discarded solids can be used to produce side products using additional refining processes, although this is not mentioned in detail.

The crude extract obtained in this way includes 70 ∼ 83% of betulin, 4 ∼ 8% of betulinic acid and residual polar pigment components. This crude extract is prepared depending on optimum extraction conditions for producing ultrapure betulin and highly-pure betulinic acid.

In the step of recrystallizing the crude extract, the crude extract is recrystallized using a mixture of polar solvents. Concretely, the crude extract is dissolved and recrystallized using the synergistic effect of the solubility of betulin in a mixture of chloroform and methanol or a mixture of chloroform and ethanol.

That is, in the step of recrystallizing the crude extract, the crude extract is dissolved in a mixture of polar organic solvents at room temperature, cooled to -5 ∼ 10 °C, and then left for 20 ∼ 30 hours (preferably, about 24 hours) to form pine needle-shaped or snowflake-shaped white crystals. Then, the white crystals are filtered using a 0.5 µm PTFE membrane and then dried to form a crystallized product.

Further, in the step of recrystallizing the crude extract, 40∼80 % of the amount of the solvent included in the filtered solution containing the crystallized product is evaporated to concentrate betulin, betulinic acid and polar pigment components in the filtered solution, the concentrated filtered solution is left at a temperature of -5 ∼ 10 °C to form a crystallized product in the solution, and the solution containing the crystallized product is filtered and dried to obtain a secondary crystallized product.

As described above, the recrystallization step may be repeated several times to obtain a tertiary crystallized product. Further, the recrystallization step is characterized in that a mixture of polar organic solvents is used. The recrystallization step is greatly advantageous in that the consumption of a solvent can be reduced by 47.3 ∼ 88.7% compared to when a raw material is dissolved in a single polar solvent, and simultaneously a high-quality crystallized product (including a secondary crystallized product) including 94 ∼ 96.5% of betulin, 3 ∼ 4% of betulinic acid and residual polar pigment components can be produced.

This recrystallization step is an intermediate step for obtaining ultrapure betulin of 99.5% or more and highly-pure betulinic acid of 95% or more. The outer bark and inner bark of a birch tree generally includes betulin, betulinic acid, lupeol (CAS 545-47-1) and polar pigment components. These components have thermodynamic properties such as polarity and the like because each of the molecules thereof has a hydroxy group (-OH) and an alcohol group ((-CH₂OH). That is, these components have thermodynamically high solubility in a polar organic solvent. For example, when a single polar organic solvent is used, the solubility of betulin in methanol is 6.73 g/L or less at 64.7°C, the solubility of betulin in chloroform is 40.69 g/L or less at 60.5°C, the solubility of betulin in ethanol is 18.49 g/L or less at 78°C, the solubility of betulin in ethyl acetate is 40.69 g/L or less at 60.5°C, and the solubility of betulin in ethanol is 23.02 g/L or less at 77.1°C. When the outer bark of a birch tree is extracted using such a polar organic solvent, a crude extract including 70 ∼ 83% of betulin, 4 ∼ 8% of betulinic acid and residual polar pigment components can be obtained. In this case, in order to use betulin and betulinic acid as intermediate materials for fine chemical products or medical supplies or additives for enhancing antiviral activity, the crude extract is recrystallized.

Preferably, a mixture of organic solvents having a density range of 0.91 g/mL ∼ 1.37 g/mL may be used. For example, a mixture of chloroform and ethanol (chloroform: ethanol = 18:82 v/v, density = 0.91 g/mL, betulin solubility = 38.6 g/L) and a mixture of chloroform and ethanol (chloroform: ethanol = 82:18 v/v, density = 1.37 g/mL, betulin solubility = 162.8 g/L) may be used. The crude extract is recrystallized using such a mixture of organic solvents to obtain a high-quality crystallized product (including a secondary crystallized product) including 94 ∼ 96.5% of betulin, 3 ∼ 4% of betulinic acid and residual polar pigment components, and then ultrapure betulin is produced using a 4-bed eluent swing continuous adsorption unit.

In the step of forming the feed solution, the crystallized product (including the secondary crystallized product) is dissolved in a single polar organic solvent or a mixture of polar organic solvents to form a feed solution for producing ultrapure betulin and highly-pure betulinic acid. In this step, a single polar organic solvent selected from ethanol, methanol, acetone and chloroform may be used, or a mixed polar organic solvent in which ethanol, methanol or acetone is mixed with chloroform may be used.

Here, the mixed polar organic solvent may be a mixture of ethanol and chloroform having a density of 0.9 ∼ 1.3 g/mL, a mixture of methanol and chloroform having a density of 0.9 ∼ 1.3 g/mL or a mixture of acetone and chloroform having a density of 0.9 ∼ 1.3 g/mL.

The step of separating the feed solution using the 4-bed eluent swing continuous adsorption unit may include the steps of: separating polar pigment components and betulinic acid from the feed solution by adsorbing them using columns to produce betulin; and supplying a regeneration solvent to each of the columns adsorbed with polar pigment components and betulinic acid to regenerate the columns, and simultaneously separating polar pigment components from the regeneration solvent discharged from the columns to produce highly-pure betulinic acid

The step of producing betulin may include the steps of: supplying the feed solution to a first column to remove polar pigment components from the feed solution by allowing the first column to adsorb the polar pigment components; supplying the feed solution discharged from the first column to a second column to remove betulinic acid from the feed solution by allowing the second column to adsorb the betulinic acid; and collecting, evaporating and drying the feed solution discharged from the second column to produce ultrapure betulin.

The step of regenerating the columns may include the steps of: supplying a regeneration solvent (methanol, ethanol, acetone or ethyl acetate) into a third column adsorbed with polar pigment components to separate the polar pigment components from a fixed bed of the third column, and collecting, evaporating and drying the feed solution discharged from the third column to obtain the polar pigment components; and supplying a regeneration solvent (methanol, ethanol, acetone or ethyl acetate) into a fourth column adsorbed with betulinic acid to separate the betulinic acid from a fixed bed of the fourth column, and collecting, evaporating and drying the feed solution discharged from the fourth column to obtain the betulinic acid.

In this case, the step of producing betulin using the first and second columns and the step of regenerating the third and fourth columns may be simultaneously performed, and each of the first, second, third and fourth columns may be charged with polar silica gel or aluminum oxide.

That is, in the step of separating the feed solution using the 4-bed eluent swing continuous adsorption unit, the feed solution is supplied to the first and second columns or the third and fourth columns by a main valve. In this case, when the production of betulin is completed by one side column line (for example, first and second columns), the feed solution is supplied to the other column line (for example, third and fourth columns) by the main valve to continuously produce betulin, and the regenerating solvent is supplied to the one side column line (for example, first and second columns) to regenerate the first and second columns. These procedures are repeated several times to continuously produce ultrapure betulin and highly-pure betulinic acid.

In this way, polar pigment components having higher polarity than betulin are adsorbed and removed by the first and third columns, betulin and betulinic acid are introduced into the second and fourth columns to allow betulinic acid having slightly higher polarity than betulin to be adsorbed and removed, and a betulin solution is obtained from the outlet.

Hereinafter, an apparatus for simultaneously producing ultrapure betulin and highly-pure betulinic acid from bark of a birch tree according to the present invention will be described in detail.

The apparatus for simultaneously producing ultrapure betulin and highly-pure betulinic acid from bark of a birch tree according to the present invention includes: first and third columns (I-A and I-B) for adsorbing and separating polar pigment components included in a feed solution; a main valve (V21) for supplying the feed solution to a supply line (11) of the first column or a supply line (31) of the third column; a second column (II-A) connected to a discharge line (12) of the first column to adsorb and separate betulinic acid from the feed solution discharged from the first column; a fourth column (II-B) connected to a discharge line (32) of the third column to adsorb and separate betulinic acid from the feed solution discharged from the third column; a betulin discharge line 20 which is connected to discharge lines 13 and 33 of the second and fourth columns, which is provided with a discharge switching valve (V25) and which discharges the betulin-containing feed solution from which the polar pigment component and betulinic acid were removed by the first, second, third and fourth columns (I-A, I-B, II-A and II-B); a regeneration solvent supply line 40 which is connected to discharge lines 12 and 32 of the first and third columns, which is provided with a second switching valve (V23) and which supplies a regeneration solvent (I) to the first column (I-A) or the third column (I-B) using the second switching valve (V23); a polar pigment component discharge line 50 which is connected to the supply lines 11 and 31 of the first and third columns, which is provided with a first switching valve (V22) and which discharges the polar pigment component-containing regeneration solvent (I) in the first and third columns (I-A and I-B) using the first switching valve (V22); a regeneration solvent supply line 60 which is connected to the discharge lines 13 and 33 of the second and fourth columns, which is provided with a fourth switching valve (V26) and which supplies a regeneration solvent (II) to the second column (II-A) or the fourth column (II-B) using the fourth switching valve (V26); and a betulinic acid discharge line 70 which is connected to the discharge lines 12 and 32 of the first and third columns, which is provided with a third switching valve (V24) and which discharges the betulinic acid-containing regeneration solvent (II) in the second and fourth columns (II-A and II-B) using the third switching valve (V24).

That is, in the apparatus, the feed solution is supplied to the first column (I-A) or the third column (I-B) by the main valve (V21), and, when the feed solution is first supplied to the first column (I-A), polar pigment components are removed by the polar fixed bed charged in the first column (I-A). Subsequently, when the feed solution from which polar pigment components were removed and which was discharged from the first column (I-A) is supplied to the second column (II-A) by the valve (V27), betulinic acid is removed by the polar fixed bed charged in the second column (II-A) . Subsequently, when the feed solution from which betulinic acid was removed and which was discharged from the second column (II-A) is discharged to the betulin discharge line by the discharge switching valve (V25), the organic solvent included in the betulin-containing feed solution discharged through the betulin discharge line is completely evaporated, thus producing ultrapure betulin having purity of 99.5% or more.

Further, after the production of betulin is completed by the first and second columns (I-A and II-A), when the feed solution is supplied to the third column (I-B) by the main valve (V21), polar pigment components are removed by the polar fixed bed charged in the third column (I-B). Subsequently, when the feed solution from which polar pigment components were removed and which was discharged from the third column (I-B) is supplied to the fourth column (II-B) by the valve (V28), betulinic acid is removed by the polar fixed bed charged in the fourth column (II-B) . Subsequently, when the feed solution from which betulinic acid was removed and which was discharged from the fourth column (II-B) is discharged to the betulin discharge line by the discharge switching valve (V25), the organic solvent included in the betulin-containing feed solution discharged through the betulin discharge line is completely evaporated, thus producing ultrapure betulin having purity of 99.5% or more.

Further, at the time of producing betulin using the third and fourth columns (I-B and II-B), when the regeneration solvent I (methanol, ethanol or acetone) is heated from room temperature to a temperature of 50 ∼ 100% of the boiling point thereof (for example, methanol is heated to 32 ∼ 64.7°C, ethanol is heated to 39 ∼ 78.4°C, and acetone is heated to room temperature ∼ boiling point) and then supplied into the first column (I-A) through the generation solvent I supply line, and when the generation solvent II, which is a pure polar solvent (methanol, ethanol, acetone or ethyl acetate), is supplied into the second column (II-A) through the generation solvent II supply line, polar pigment components are separated from the first column (I-A) and betulinic acid is separated from the second column (II-A) to respectively regenerate the first and second columns (I-A and II-A), and the polar pigment component-containing feed solution discharged from the first column (I-A) is discharged to the polar pigment component discharge line by the valve (V22) and then completely evaporated to obtain polar pigment components.

Further, the betulinic acid-containing feed solution discharged from the second column (II-A) is discharged to the betulinic acid discharge line by the valve (V24) and then completely evaporated to obtain betulinic acid.

As such, betulin and betulinic acid can be continuously produced by repeatedly alternating the first and second columns with the third and fourth columns. Further, the solvent recovered by condensation is reused to regenerate the second column (II-A) and the fourth column (II-B).

### Mode for Invention

Hereinafter, the present invention will be described in more detail with reference to the following examples.

### Example 1

HPLC analysis (calibration curve) of betulin and betulinic acid

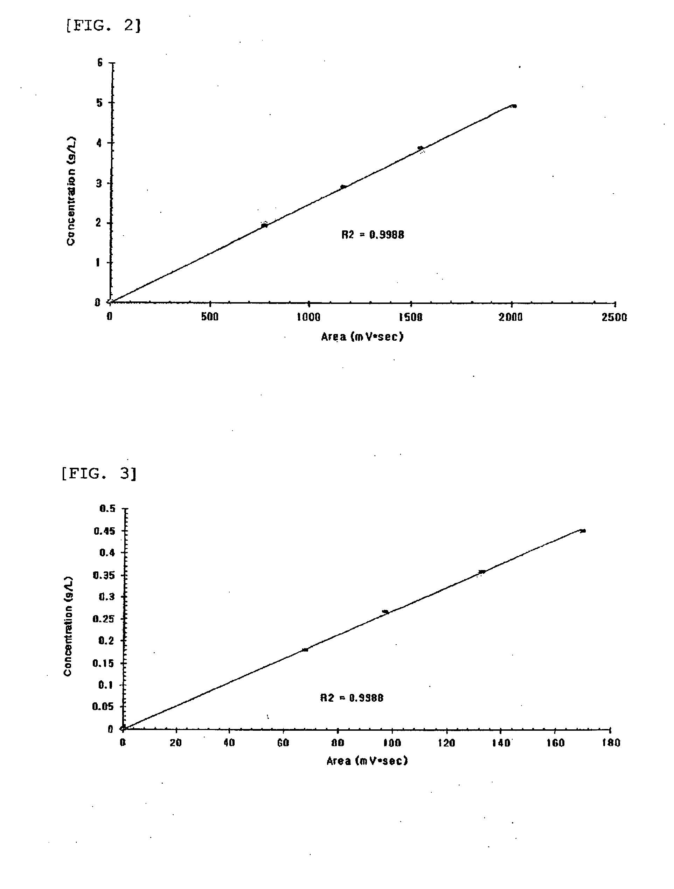

The concentrations of a crude extract, a primarily crystallized product, ultrapure betulin and highly-pure betulinic acid were quantitatively analyzed using the calibration curves of FIGS. 2 and 3. Betulin (Sigma: B8795, minimum 98%) and betulinic acid (Sigma: 855057, minimum 90%) were used as standard samples. 100 mg of betulin (standard sample) was dissolved in 20 mL of HPLC-grade ethanol to prepare a mother solution having a concentration of 5.0 g/L, the mother solution was diluted to form reagents having concentrations of 0.4 g/L, 0.3 g/L and 0.2 g/L, and then the reagents were analyzed using HPLC. 10 mg of betulinic acid (standard sample) was dissolved in 20 mL of HPLC-grade ethanol to prepare a mother solution having a concentration of 0.5 g/L, the mother solution was diluted to form reagents having concentrations of 0.4 g/L, 0.3 g/L and 0.2 g/L, and then the reagents were analyzed using HPLC [HPLC Column: Nova-Pak C18-ODS reverse phase, 4 µm, 3.9x300 mm, eluent: CH₃CN:H₂O=86:14 v/v]. The concentration of betulin included in an unknown reagent was determined using the calibration curve made by plotting the area of the HPLC peak obtained by analyzing each of the samples with concentration and then conducting a linear regression analysis.

### Example 2

### Production of a crude betulin extract from betula papyrifera using ethyl acetate

The inner and outer barks of *betula papyrifera* naturally growing in the North America were dried to have a moisture content of 6% and pulverized into particles having a size of 8 mm or less, 150 g of the particles and 1900 mL of ethyl acetate were charged in a reflux extractor (12.67 L per kg of outer bark), and then the mixture was heated for 1 hour at 62°C and then for 3 hours at 77°C under atmospheric pressure to extract betulin and betulinic acid into the organic solvent. The mixture charged in the reflux extractor was hot-filtered at 70°C using a 0.5 µm PTFE membrane to separate the organic solvent from the wet bark while preventing the congelation of betulin. 80% of the total volume of the filtered solution was evaporated to concentrate the filtered solution, and then the concentrated solution was left for 10 hours at -2°C to crystallize betulin. The mixture of the crystallized betulin and ethyl acetate was filtered using the 0.5 µm PTFE membrane to obtain a betulin-crystallized product, and then the betulin-crystallized product was dried at 35°C to obtain 12 g of a solid (primary crude extract). Further, 80% of the total volume of the filtered solution was evaporated to concentrate the filtered solution, the concentrated solution was left for 4 hours at -2°C to crystallize betulin, the mixture of the crystallized betulin and ethyl acetate was filtered using the 0.5 µm PTFE membrane to obtain a betulin-crystallized product, and then the betulin-crystallized product was dried at 35°C to obtain 2.7 g of a solid (secondary crude extract).

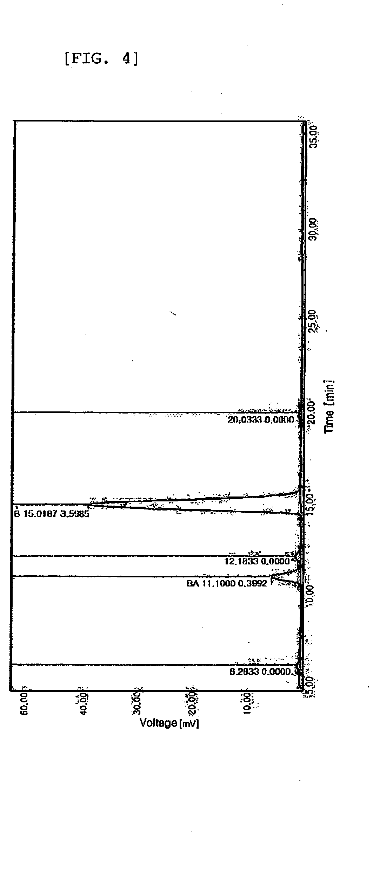

The yield of the crude extract was 9.8% (dried outer bark weight / crude extract weight x 100 = 14.7/150 x 100). FIG. 4 shows the results of HPLC analysis of the sample made by dissolving 100 mg of betulin (standard sample) in 20 mL of HPLC-grade ethanol. As shown in FIG. 4, the crude extract included 75 % betulin (retention time: 15.0 minutes), 4% of betulinic acid (retention time: 11.1 minutes), and 21% of unidentified polar pigment components (retention time: 6.26 minutes, 12.2 minutes, and 20.03 minutes).

### Example 3

### Production of a crude betulin extract from betula papyrifera using chloroform

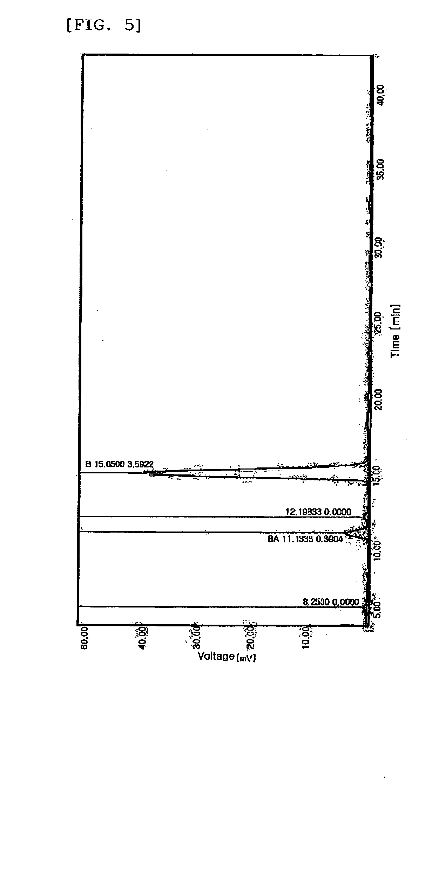

178 g of the inner and outer barks (having a particle size of 8 mm or less and a moisture content of 5%) of *betula papyrifera* naturally growing in the North America were put into a reflux extractor charged with 1900 mL of chloroform (10.67 L per kg of outer bark). The mixture was heated for 1 hour at 49°C and then for 2 hours at 61.2°C to extract betulin and betulinic acid into the organic solvent. The mixture charged in the reflux extractor was filtered at a temperature near the boiling point of chloroform in order to prevent the congelation of betulin. The organic solvent and extracts were separated from the bark, 80% of the total volume of the filtered solution was evaporated to concentrate the filtered solution, and then the concentrated solution was cooled at room temperature and then left for 10 hours at -2°C to primarily crystallize betulin and betulinic acid. The crystallized product was filtered using a 0.5 µm PTFE membrane to obtain a solid crystallized product, and then the solid crystallized product was dried at 35°C to obtain a primary crude extract. Further, 80% of the total amount of the organic solvent was evaporated from the filtered solution to concentrate the filtered solution, and then the concentrated solution was left for 3 hours at -2°C to secondarily crystallize betulin and betulinic acid. The crystallized product was filtered and dried in the same manner as above to obtain a solid crystallized product, and then the solid crystallized product was dried in the same manner as above to obtain a secondary crude extract. A sample was made in the same manner as in Example 2, and the concentration of the sample was analyzed using HPLC. The results thereof are shown in FIG. 5.

18.8 g of the crude extract was obtained, and the yield of the crude extract was 10.7%. The crude extract included 70.44% betulin, 5.89% of betulinic acid, and residual unknown polar pigment components (retention time: 6.25 minutes and 12.2 minutes).

### Example 4

### Production of a crude betulin extract from betula papyrifera using a mixed solvent of chloroform and ethanol

165 g of bark particles of *betula papyrifera* were put into a reflux extractor charged with 1900 mL of a mixed solvent (density: 1.3 g/mL or less) of chloroform and ethanol. The mixture was heated for 1 hour at 63°C and then for 3 hours at 78.4°C (11.5 L per kg of outer bark) to extract betulin and betulinic acid into the organic solvent. The mixed solution was filtered to separate betulin and betulinic acid from the bark thereof, and 80% of the total amount of the filtered solution was evaporated to concentrate the filtered solution, and then the concentrated solution was left for 10 hours at - 2°C to primarily crystallize betulin. The crystallized product was filtered using a 0.5 µm PTFE membrane to obtain a solid crystallized product, and then the solid crystallized product was dried at 35°C to obtain a primary crude extract. Further, 80% of the total amount of the organic solvent was evaporated from the filtered solution to concentrate the filtered solution, and then the concentrated solution was left for 3 hours at -2°C to secondarily crystallize betulin.

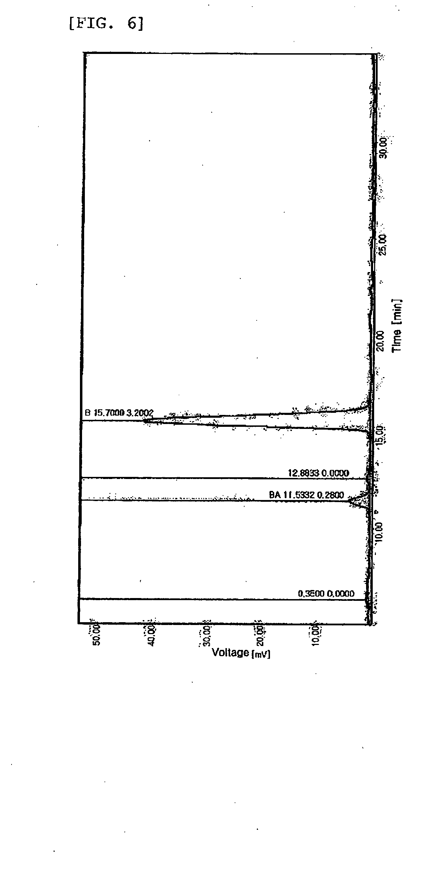

25.2 g of the crude extract was obtained, and the yield thereof was 15.2%. A sample was made in the same manner as in Example 2, and the concentration of the sample was analyzed using HPLC. The results thereof are shown in FIG. 6. As shown in FIG. 6, the crude extract included 76.87% betulin, 5.49% of betulinic acid, and residual unknown polar pigment components (retention time: 6.25 minutes and 12.2 minutes).

### Example 5

### Production of a crude betulin extract from betula platyphylla using ethyl acetate

263.8 g of inner and outer bark particles (having a particle size of 8 mm or less and a moisture content of 6%) of *betula platyphylla* naturally growing in the Korea were put into a reflux extractor charged with 1880 mL of ethyl acetate (bark weight (kg): organic solvent (L) = 7.13: 1). The mixture was heated for 1 hour at 62°C and then for 3 hours at 77°C to extract betulin (main component) and betulinic acid (side component) into the organic solvent. The mixed organic solvent and extracts were separated from the wet bark, and were then used in the following secondary extraction. 80 % of the amount of the filtered solution was evaporated to concentrate the filtered solution, and then the concentrated solution was left for 10 hours at -2°C to primarily crystallize betulin and betulinic acid. The crystallized product was filtered using a 0.5 µm PTFE membrane to obtain a solid crystallized product, and then the solid crystallized product was dried at 35°C to obtain a primary crude extract. Subsequently, 70% of the amount of the organic solvent was evaporated from the filtered solution to concentrate the filtered solution, and then the concentrated solution was left for 4 hours at -2°C to secondarily crystallize betulin and betulinic acid. The crystallized product was filtered using a 0.5 µm PTFE membrane to obtain a solid crystallized product, and then the solid crystallized product was dried at 35°C to obtain a primary crude extract. 1150 mL of ethyl acetate was put into the reflux extractor charged with the wet bark (extracted residue), the mixed solution was heated at 77.1°C for 30 minutes and filtered by the 0.5 µm PTFE membrane, and the filtered solution was crystallized and dried at 35 °C to obtain a tertiary crude extrude.

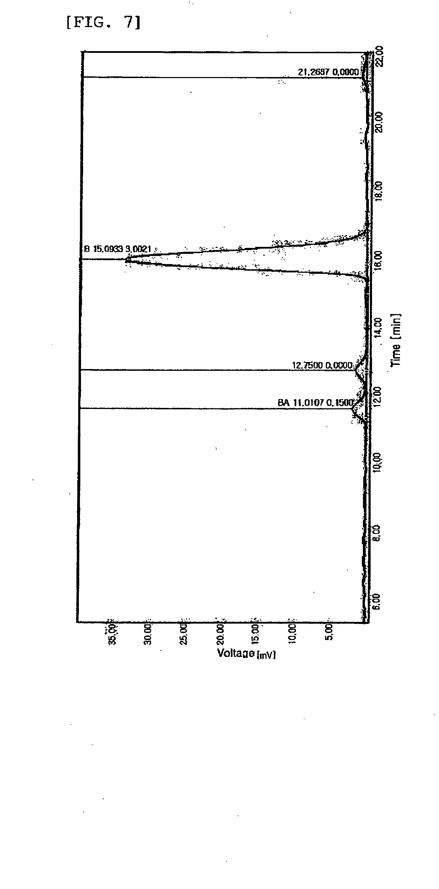

59.5 g of the crude extract was obtained, and the yield thereof was 22.5%. A sample was made in the same manner as in Example 2, and the concentration of the sample was analyzed using HPLC. The results thereof are shown in FIG. 7. As shown in FIG. 7, the crude extract included 82.46% betulin, 4.18% of betulinic acid, and residual unknown polar pigment components (retention time: 12.75 minutes and 21.26 minutes).

### Example 6

### Recrystallization of a crude extract using a mixed solvent of chloroform and ethanol

The crude extract obtained from Example 5 was put into a crystallization container charged with 343 mL of a mixed solvent (specific gravity: 1.367 g/mL) of chloroform and ethanol and dissolved in the mixed solvent. Subsequently, the mixed solution was slowly cooled to 20°C for 2 hours, and then left at 2°C for 24 hours to form pine needle-shaped white crystals in the mixed solution. The mixed solution containing the white crystals was filtered and then dried to obtain primary crystals (21.51 g, yield: 37%). 80% of the filtered mixed solution was evaporated, recrystallized at -2°C, filtered and then dried to obtain secondary crystals (16 g, yield: 27.6%).

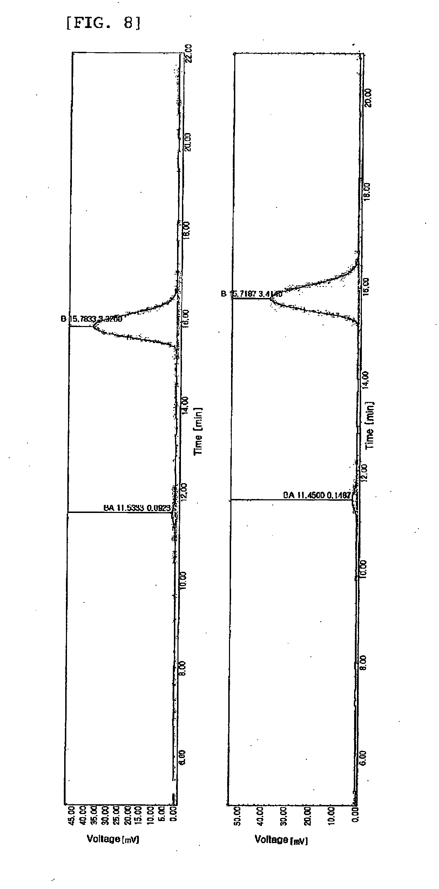

The primary crystals were analyzed using HPLC in the same manner as in Example 2, and the results thereof are shown in FIG. 8-A. As shown in FIG. 8-A, the content of betulin was 97.29%, and the content of betulinic acid was 2.71%. The secondary crystals were analyzed using HPLC in the same manner as in Example 2, and the results thereof are shown in FIG. 8-B. As shown in FIG. 8-B, the content of betulin was 95.9%, and the content of betulinic acid was 4.17%.

### Example 7

### Simultaneous production of ultrapure betulin and highly-pure betulinic acid using 4-bed eluent swing continuous adsorption unit

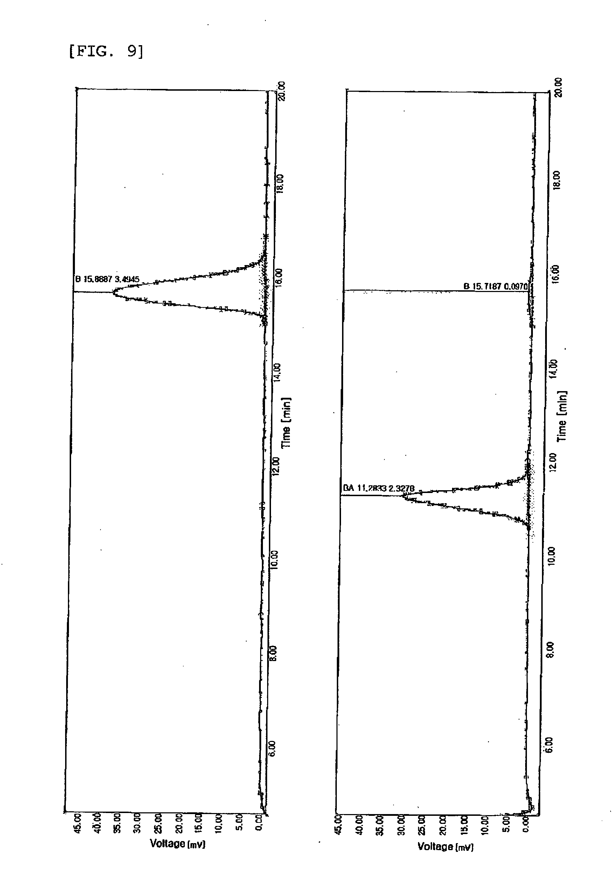

35.14 g of a crude extract including 96.03% of betulin and 3.97% of betulinic acid was dissolved in 1100 mL of a mixed solvent (chloroform: ethanol = 3:2) to prepare a feed solution. The feed solution was supplied to the top of a first column (I-A) at a flow rate of 2.5 mL/min to remove polar pigment components using adsorption, and the feed solution discharged from the first column (I-A) was supplied to a second column (II-A) to remove betulinic acid using adsorption. The feed solution discharged from the second column (II-A) was collected, evaporated and dried to obtain 31.52 g of white crystals (yield: 89.7%) including 99.8% of betulin and 0.2% of betulinic acid (refer to FIG. 9-A). In order to desorb polar pigment components, 500 mL of methanol was heated to 60°C and then supplied to a third column (II-A). In this case, the feed solution discharged from the third column (IT-A) was evaporated and dried to remove the obtained polar pigment components. 600 mL of ethyl acetate was supplied to a fourth column (II-B) to desorb betulinic acid, and the feed solution discharged from the fourth column (II-B) was evaporated and dried to obtain 0.584 g of betulinic acid having purity of 96% (refer to FIG. 9-B).

The molecular structures of betulin and betulinic acid were respectively ascertained using nuclear magnetic resonance spectrum analysis (¹³C NMR (125 MHz, CDCl₃), ¹H NMR (600 MHz, CDCl₃)). In this case, the third and fourth columns (II-A and II-B) are respectively adsorbed with polar pigment components and betulinic acid because they were previously used to produce betulin.

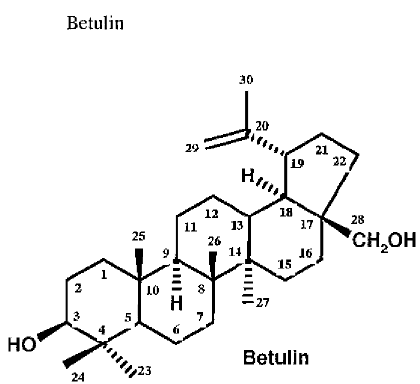

| ¹³C NMR (125 MHz, CDCl₃) | | | | | | | | | |
|---|---|---|---|---|---|---|---|---|---|
| **C1** | 38.890 | **C2** | 27.581 | **C3** | 79.179 | **C4** | 39.059 | **C5** | 55.469 |
| **C6** | 18.493 | **C7** | 34.412 | **C8** | 41.104 | **C9** | 50.581 | **C10** | 37.340 |
| **C11** | 21.014 | **C12** | 25.390 | **C13** | 37.490 | **C14** | 42.907 | **C15** | 27.230 |
| **C16** | 29.355 | **C17** | 47.979 | **C18** | 48.939 | **C19** | 48.000 | **C20** | 150.676 |
| **C21** | 29.927 | **C22** | 34.163 | **C23** | 28.182 | **C24** | 15.561 | **C25** | 16.309 |
| **C26** | 16.170 | **C27** | 14.953 | **C28** | 60.746 | **C29** | 109.896 | **C30** | 19.210 |

¹H NMR (600 MHz, CDCl₃):
0.88 (1H, m, Hₐ-1), 1.64 (1H, m, H_{b}-1), 1.54 (1H, m, Hₐ-2), 1.58 (1H, m, H_{b}-2), 3.19 (1H, dd, J=11.1, 5.58 Hz, H-3), 0.68 (1H, m, H-5), 1.53 (1H, m, Hₐ-6), 1.37 (1H, m, H_{b}-6), 1.38(2H, m, H-7), 1,26 (1H, m, H-9), 1.42 (1H, m, Hₐ-11), 1.23 (1H, m, H_{b}-11), 1.63 (1H, m, Hₐ-12), 1.01 (1H, m, H_{b}-12), 1.61 (1H, m, H-13), 1.67 (1H, m, Hₐ-15), 1.04 (1H, m, H_{b}-15), 1.92 (1H, m, Hₐ-16), 1.19 (1H, m, H_{b}-16), 1.56 (1H, m, H-18), 2.38 (1H, ddd, J=5.7, 11 Hz, H-19), 1.92 (1H, m, Hₐ-21), 1.42 (1H. M, H_{b}-21), 1.04 (1H, m, Hₐ-22), 1.85 (1H, m, H_{b}-22), 0.97 (3H, s, H-23), 0.76 (3H, s, H-24), 0.83 (3H, s, H-25), 1.02 (3H, s, H-26), 0.98 (3H, s, H-27), 3.80 (1H, dd, J= 10.7, 4,3 Hz, Hₐ-28), 3.32 (1H, dd, J=10.7, 3.7 Hz, H_{b}-28), 4.68 (1H, s, Hₐ-29), 4.58 (1H, s, H_{b}-29), 1.68 (3H, s, H-30). 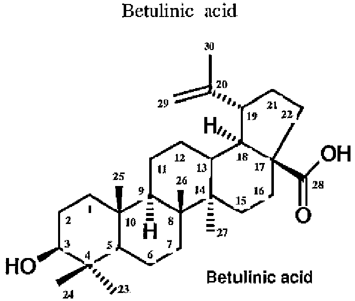
¹H NMR (600 MHz, C₅D₅N):
δ=4.95 (1H, s, H-29a), 4.79 (1H. s, II-29b), 3.47-3.54 (1H, m, H-3), 1.80 (3H, s, H-30), 1.41-1.32 (3H, m, H-9, H-12); 1.26 (3H, s, H-27), 1.24 (3H, s, H-26), 1.06 (3H, s, H-23), 1.05 (3H, s, H-27), 1,03 (3H, s, H-25), 0.83 (3H, s, H-25)
¹³C NMR (125 MHz, C₅D₅H):
   δ=179.22 (C28), 151.59 (C20), 110.25 (C30), 78.44 (C3), 56.89 (C17), 56.17 (C5), 51.63 (C9), 51.19 (C19), 47.94 (C18), 42.68 (C14), 41.20 (C8), 39.76 (C1), 39.54 (C4), 38.85 (C13), 37.85 (C10), 37.74 (C22), 35.07 (C7), 33.12 (C16), 31.43 (C15), 30.52 (C21), 28.93 (C23), 28.71 (C2), 26.36 (C12), 21.45 (C11), 19.73 (C29), 19. 02 (C6), 16.67 (C25), 16.62 (C24), 15.97 (C26), 15.16 (C27).
¹H and ¹³C-NMR spectra of our sample are in good agreement with those previously published for betulinic acid.

## Claims

1. A method of simultaneously producing ultrapure betulin and highly-pure betulinic acid from bark of a birch tree, comprising the steps of:
preparing a crude extract from bark of a birch tree pulverized with a single polar organic solvent selected from ethanol, methanol, chloroform and ethyl acetate, or a mixed polar organic solvent thereof, by primary reflux extracting, filtering, crystallizing at a temperature of from -5 to 10 °C, filtering and drying, wherein the crude extract comprises 70∼83% of betulin, 4∼8% of betulinic acid, and residual polar pigment components;
crystallizing the crude extract at a temperature of from -5 to 10 °C, by extracting the crude extract using a mixed polar organic solvent selected from the mixtures chloroform/methanol and chloroform/ethanol, and then filtering and drying the crude extract, so as to obtain a crystallized product;
dissolving the crystallized product in a single polar organic solvent selected from ethanol, methanol, acetone and chloroform or a mixed polar organic solvent selected from the mixture of chloroform with ethanol, methanol or acetone, to form a feed solution; and
separating the feed solution using a 4-bed eluent swing continuous adsorption process to simultaneously produce ultrapure betulin having purity of 99.5% or more and highly-pure betulinic acid having purity of 95% or more.

2. The method of claim 1, wherein, in the step of crystallizing the crude extract, the crystallized product comprises 94∼96.5% of betulin, 3∼4% of betulinic acid, and residual polar pigment components.

3. The method of claim 1, wherein the step of crystallizing the crude extract comprises the steps of:
evaporating 40∼80% of a solvent from a solution containing the crude extract to concentrate betulin, betulinic acid, and polar pigment components in the solution;
leaving the solution at a temperature of -5∼10 °C to prepare a primarily crystallized product in the solution; and
filtering and drying the solution containing the primarily crystallized product to obtain a secondarily crystallized product.

4. The method of claim 1, wherein, in the step of forming the feed solution, the single polar organic solvent is any one selected from ethanol, methanol, acetone and chloroform, and the mixed polar organic solvent is a mixture of ethanol, methanol or acetone and chloroform and has a density of 0.91∼1.37 g/mL.

5. The method of claim 1, wherein the step of separating the feed solution using the 4-bed eluent swing continuous adsorption process comprises the steps of:
separating polar pigment components and betulinic acid from the feed solution by adsorbing them using columns to produce betulin; and
supplying a regeneration solvent to each of the columns adsorbed with polar pigment components and betulinic acid to regenerate the columns, and simultaneously separating polar pigment components from the regeneration solvent discharged from the columns to produce highly-pure betulinic acid,
wherein the step of producing betulin and the step of regenerating the columns are simultaneously performed.

6. The method of claim 5, wherein the step of producing betulin comprises the steps of:
supplying the feed solution to a first column to remove polar pigment components from the feed solution by allowing the first column to adsorb the polar pigment components;
supplying the feed solution discharged from the first column to a second column to remove betulinic acid from the feed solution by allowing the second column to adsorb the betulinic acid; and
collecting, evaporating and drying the feed solution discharged from the second column to produce ultrapure betulin.

7. The method of claim 5, wherein the step of regenerating the columns comprises the steps of:
supplying a regeneration solvent (methanol, ethanol, acetone, or ethyl acetate) into a third column adsorbed with polar pigment components to separate the polar pigment components from a fixed bed of the third column, and collecting, evaporating, and drying the feed solution discharged from the third column to obtain the polar pigment components; and
supplying a regeneration solvent (methanol, ethanol, acetone, or ethyl acetate) into a fourth column adsorbed with betulinic acid to separate the betulinic acid from a fixed bed of the fourth column, and collecting, evaporating, and drying the feed solution discharged from the fourth column to obtain the betulinic acid.

8. An apparatus for simultaneously producing ultrapure betulin, and highly-pure betulinic acid from bark of a birch tree, comprising:
first and third columns (I-A and I-B) for adsorbing and separating polar pigment components included in a feed solution;
a main valve (V21) for supplying the feed solution to a supply line (11) of the first column or a supply line (31) of the third column;
a second column (II-A) connected to a discharge line (12) of the first column to adsorb and separate betulinic acid from the feed solution discharged from the first column;
a fourth column connected to a discharge line (32) of the third column to adsorb and separate betulinic acid from the feed solution discharged from the third column;
a betulin discharge line (20) which is connected to discharge lines (13) and (33) of the second and fourth columns, which is provided with a discharge switching valve (V25) and which discharges the betulin-containing feed solution from which the polar pigment component and betulinic acid were removed by the first, second, third and fourth columns (I-A, I-B, II-A and II-B);
a regeneration solvent supply line (40) which is connected to discharge lines of the first and third columns, which is provided with a second switching valve (V23) and which supplies a first regeneration solvent (I) to the first column (I-A) or the third column (I-B) using the second switching valve (V23);
a polar pigment component discharge line (50) which is connected to the supply lines (11) and (31) of the first and third columns, which is provided with a first switching valve (V22) and which discharges the first polar pigment component-containing regeneration solvent (I) in the first and third columns (I-A) and (I-B) using the first switching valve (V22);
a regeneration solvent supply line (60) which is connected to the discharge lines (13) and (33) of the second and fourth columns, which is provided with a fourth switching valve (V26) and which supplies a second regeneration solvent (II) to the second column (II-A) or the fourth column (II-B) using the fourth switching valve (V26); and
a betulinic acid discharge line (70) which is connected to the discharge lines(12) and (32) of the first and third columns, which is provided with a third switching valve (V24) and which discharges the second betulinic acid-containing regeneration solvent (II) in the second and fourth columns (II-A) and (II-B) using the third switching valve (V24).

## Patentansprüche

1. Verfahren zur gleichzeitigen Herstellung von ultrareinem Betulin und hochreiner Betulinsäure aus Birkenrinde, mit den folgenden Schritten:
Herstellen eines Rohextrakts aus pulverisierter Birkenrinde mit einem einzigen polaren organischen Lösungsmittel, das aus Ethanol, Methanol, Chloroform und Ethylacetat ausgewählt ist, oder einem gemischten polaren organischen Lösungsmittel davon, indem zunächst bei einer Temperatur von -5 bis 10 °C unter Rückfluss extrahiert, gefiltert und kristallisiert wird, dann gefiltert und getrocknet wird, wobei der Rohextrakt 70∼83% Betulin, 4∼8% Betulinsäure und restliche polare Pigmentbestandteile umfasst;
Kristallisieren des Rohextrakts bei einer Temperatur von -5 bis 10 °C, indem der Rohextrakt unter Verwendung eines gemischten polaren organischen Lösungsmittels, das aus den Mischungen Chloroform/Methanol und Chloroform/Ethanol ausgewählt ist, extrahiert wird und der Rohextrakt dann gefiltert und getrocknet wird, um ein kristallisiertes Produkt zu erhalten;
Lösen des kristallisierten Produkts in einem einzigen polaren organischen Lösungsmittel, das aus Ethanol, Methanol, Aceton und Chloroform ausgewählt ist, oder in einem gemischten polaren organischen Lösungsmittel, das aus der Mischung von Chloroform mit Ethanol, Methanol oder Aceton ausgewählt ist, um eine Einsatzlösung zu bilden; und
Trennen der Einsatzlösung unter Verwendung eines kontinuierlichen, 4-Bett-Eluentenwechseladsorptionsverfahrens, um gleichzeitig ultrareines Betulin mit einer Reinheit von 99,5% oder mehr und hochreine Betulinsäure mit einer Reinheit von 95% oder mehr herzustellen.

2. Verfahren nach Anspruch 1, wobei bei dem Schritt des Kristallisierens des Rohextrakts das kristallisierte Produkt 94∼96,5% Betulin, 3∼4% Betulinsäure und restliche polare Pigmentbestandteile umfasst.

3. Verfahren nach Anspruch 1, wobei der Schritt des Kristallisierens des Rohextrakts die folgenden Schritte umfasst:
Verdampfen von 40∼80% eines Lösungsmittels aus einer den Rohextrakt enthaltenden Lösung, um Betulin, Betulinsäure und polare Pigmentbestandteile in der Lösung zu konzentrieren;
Belassen der Lösung bei einer Temperatur von -5∼10 °C, um ein primär kristallisiertes Produkt in der Lösung herzustellen; und
Filtern und Trocknen der das primär kristallisierte Produkt enthaltenden Lösung, um ein sekundär kristallisiertes Produkt zu erhalten.

4. Verfahren nach Anspruch 1, wobei bei dem Schritt des Bildens der Einsatzlösung das einzige polare organische Lösungsmittel ein aus Ethanol, Methanol, Aceton und Chloroform ausgewähltes Lösungsmittel ist, und das gemischte polare organische Lösungsmittel eine Mischung von Ethanol, Methanol oder Aceton und Chloroform ist und eine Dichte von 0,91∼1,37 g/ml hat.

5. Verfahren nach Anspruch 1, wobei der Schritt des Trennens der Einsatzlösung unter Verwendung des kontinuierlichen 4-Bett-Eluentenwechseladsorptionsverfahrens die folgenden Schritte umfasst:
Abscheiden von polaren Pigmentbestandteilen und Betulinsäure aus der Einsatzlösung, indem sie mit Hilfe von Säulen adsorbiert werden, um Betulin herzustellen; und
Zuführen eines Regenerationslösungsmittels zu jeder der mit polaren Pigmentbestandteilen und Betulinsäure adsorbierten Säulen, um die Säulen zu regenerieren, und gleichzeitig Abscheiden von polaren Pigmentbestandteilen aus dem aus den Säulen ausgeleiteten Regenerationslösungsmittel, um hochreine Betulinsäure herzustellen,
wobei der Schritt der Herstellung von Betulin und der Schritt des Regenerierens der Säulen gleichzeitig durchgeführt werden.

6. Verfahren nach Anspruch 5, wobei der Schritt der Herstellung von Betulin die folgenden Schritte umfasst:
Zuführen der Einsatzlösung zu einer ersten Säule, um polare Pigmentbestandteile aus der Einsatzlösung zu entfernen, indem man die erste Säule die polaren Pigmentbestandteile adsorbieren lässt;
Zuführen der aus der ersten Säule ausgeleiteten Einsatzlösung zu einer zweiten Säule, um Betulinsäure aus der Einsatzlösung zu entfernen, indem man die zweite Säule die Betulinsäure adsorbieren lässt; und
Sammeln, Verdampfen und Trocknen der aus der zweiten Säule ausgeleiteten Einsatzlösung, um ultrareine Betulin herzustellen.

7. Verfahren nach Anspruch 5, wobei der Schritt des Regenerierens der Säulen die folgenden Schritte umfasst:
Einleiten eines Regenerationslösungsmittels (Methanol, Ethanol, Aceton oder Ethylacetat) in eine mit polaren Pigmentbestandteilen adsorbierte dritte Säule, um die polaren Pigmentbestandteile aus einem Festbett der dritten Säule abzuscheiden, und Sammeln, Verdampfen und Trocknen der aus der dritten Säule ausgeleiteten Einsatzlösung, um die polaren Pigmentbestandteile zu erhalten; und
Einleiten eines Regenerationslösungsmittels (Methanol, Ethanol, Aceton oder Ethylacetat) in eine mit Betulinsäure adsorbierte vierte Säule, um die Betulinsäure aus einem Festbett der vierten Säule abzuscheiden, und Sammeln, Verdampfen und Trocknen der aus der vierten Säule ausgeleiteten Einsatzlösung, um die Betulinsäure zu erhalten.

8. Vorrichtung zur gleichzeitigen Herstellung von ultrareinem Betulin und hochreiner Betulinsäure aus Birkenrinde, umfassend:
eine erste und eine dritte Säule (I-A und I-B) zum Adsorbieren und Abscheiden polarer Pigmentbestandteile, die in einer Einsatzlösung enthalten sind;
ein Hauptventil (V21) zum Zuführen der Einsatzlösung zu einer Zuführleitung (11) der ersten Säule oder einer Zuführleitung (31) der dritten Säule;
eine zweite Säule (II-A), die mit einer Auslassleitung (12) der ersten Säule verbunden ist, um Betulinsäure aus der aus der ersten Säule ausgeleiteten Einsatzlösung zu adsorbieren und abzuscheiden;
eine vierte Säule, die mit einer Auslassleitung (32) der dritten Säule verbunden ist, um Betulinsäure aus der aus der dritten Säule ausgeleiteten Einsatzlösung zu adsorbieren und abzuscheiden;
eine Betulinauslassleitung (20), die mit den Auslassleitungen (13) und (33) der zweiten und vierten Säule verbunden ist, mit einem Auslassumschaltventil (V25) versehen ist und die betulinhaltige Einsatzlösung ausleitet, aus der die polaren Pigmentbestandteile und Betulinsäure über die erste, zweite, dritte und vierte Säule (I-A, I-B, II-A und II-B) entfernt wurden;
eine Zuführleitung (40) für Regenerationslösungsmittel, die mit Auslassleitungen der ersten und dritten Säule verbunden ist, mit einem zweiten Umschaltventil (V23) versehen ist und ein erstes Regenerationslösungsmittel (I) unter Verwendung des zweiten Umschaltventils (V23) der ersten Säule (I-A) oder der dritten Säule (I-B) zuführt;
eine Auslassleitung (50) für polare Pigmentbestandteile, die mit den Zuführleitungen (11) und (31) der ersten und dritten Säule verbunden ist, mit einem ersten Umschaltventil (V22) versehen ist und das erste polare Pigmentbestandteile enthaltende Regenerationslösungsmittel (I) unter Verwendung des ersten Umschaltventils (V22) aus der ersten und dritten Säule (I-A) und (I-B) ausleitet;
eine Zuführleitung (60) für Regenerationslösungsmittel, die mit den Auslassleitungen (13) und (33) der zweiten und vierten Säule verbunden ist, mit einem vierten Umschaltventil (V26) versehen ist und der zweiten Säule (II-A) oder der vierten Säule (II-B) unter Verwendung des vierten Umschaltventils (V26) ein zweites Regenerationslösungsmittel (II) zuführt; und
eine Auslassleitung (70) für Betulinsäure, die mit den Auslassleitungen (12) und (32) der ersten und dritten Säule verbunden ist, mit einem dritten Umschaltventil (V24) versehen ist und das zweite, Betulinsäure enthaltende Regenerationslösungsmittel (II) in der zweiten und vierten Säule (II-A) und (II-B) unter Verwendung des dritten Umschaltventils (V24) ausleitet.

## Revendications

1. Procédé permettant de produire simultanément de la bétuline ultra-pure et de l'acide bétulinique de haute pureté à partir d'écorce de bouleau, lequel procédé comporte les étapes suivantes :
- préparer un extrait brut d'écorce de bouleau réduite en poudre, au moyen d'un seul solvant organique polaire, choisi parmi de l'éthanol, du méthanol, du chloroforme et de l'acétate d'éthyle, ou d'un mélange de ces solvants organiques polaires, par extraction primaire à reflux, filtration, cristallisation à une température de -5 à 10 °C, filtration et séchage, lequel extrait brut comprend de 70 à 83 % de bétuline et de 4 à 8 % d'acide bétulinique, ainsi que des résidus de pigments polaires ;
- faire cristalliser cet extrait brut à une température de -5 à 10 °C, en soumettant l'extrait brut à une extraction réalisée au moyen d'un mélange de solvants organiques polaires choisi parmi les mélanges chloroforme-méthanol et chloroforme-éthanol, et en récupérant ensuite l'extrait brut par filtration et en le faisant sécher, de manière à obtenir un produit cristallisé ;
- dissoudre ce produit cristallisé dans un seul solvant organique polaire, choisi parmi de l'éthanol, du méthanol, du chloroforme et de l'acétone, ou dans un mélange de solvants organiques polaires choisi parmi les mélanges de chloroforme et d'éthanol, de méthanol ou d'acétone, pour en faire une solution d'alimentation ;
- et soumettre cette solution d'alimentation à une séparation, réalisée selon un procédé d'adsorption en continu sur 4 lits avec commutation d'éluant, de manière à produire simultanément de la bétuline ultra-pure, de pureté supérieure ou égale à 99,5 %, et de l'acide bétulinique de haute pureté, pur à 95 % ou plus.

2. Procédé conforme à la revendication 1, dans lequel le produit cristallisé obtenu dans l'étape de cristallisation de l'extrait brut comprend de 94 à 96,5 % de bétuline et de 3 à 4 % d'acide bétulinique, ainsi que des résidus de pigments polaires.

3. Procédé conforme à la revendication 1, dans lequel l'étape de cristallisation de l'extrait brut comporte les opérations suivantes :
- évaporer de 40 à 80 % du solvant d'une solution contenant l'extrait brut, pour obtenir une solution concentrée de bétuline, d'acide bétulinique et de pigments polaires ;
- abandonner la solution à une température de -5 à 10 °C, pour qu'il se forme dans la solution un produit de cristallisation primaire ;
- et filtrer la solution contenant le produit de cristallisation primaire, puis la sécher pour obtenir un produit de cristallisation secondaire.

4. Procédé conforme à la revendication 1, dans lequel, dans l'étape de préparation d'une solution d'alimentation, le solvant organique polaire utilisé seul est un solvant choisi parmi de l'éthanol, du méthanol, de l'acétone et du chloroforme, et le mélange de solvants organiques polaires est un mélange de chloroforme et d'éthanol, de méthanol ou d'acétone, qui présente une masse volumique valant de 0,91 à 1,37 g/mL.

5. Procédé conforme à la revendication 1, dans lequel l'étape de séparation de la solution d'alimentation selon un procédé d'adsorption en continu sur 4 lits avec commutation d'éluant comporte les étapes suivantes :
- séparer de la solution d'alimentation les pigments polaires et l'acide bétulinique, en faisant s'adsorber ceux-ci dans des colonnes, de manière à produire de la bétuline,
- et envoyer un solvant de régénération dans chacune des colonnes où sont adsorbés les pigments polaires et l'acide bétulinique, pour régénérer ces colonnes, et en même temps, séparer les pigments polaires du solvant de régénération déchargé des colonnes, afin de produire de l'acide bétulinique de haute pureté,
étant entendu que l'opération de production de bétuline et l'opération de régénération des colonnes sont effectuées simultanément.

6. Procédé conforme à la revendication 5, dans lequel l'étape de production de bétuline comporte les opérations suivantes :
- envoyer la solution d'alimentation dans une première colonne, pour séparer les pigments polaires de la solution d'alimentation en laissant les pigments polaires s'adsorber sur cette première colonne,
- envoyer la solution d'alimentation déchargée de la première colonne dans une deuxième colonne, pour séparer l'acide bétulinique de la solution d'alimentation, en laissant l'acide bétulinique s'adsorber sur cette deuxième colonne,
- et recueillir la solution d'alimentation déchargée de la deuxième colonne et l'évaporer à sec, de manière à produire de la bétuline ultra-pure.

7. Procédé conforme à la revendication 5, dans lequel l'étape de régénération des colonnes comporte les opérations suivantes :
- envoyer un solvant de régénération (méthanol, éthanol, acétone ou acétate d'éthyle) dans une troisième colonne où sont adsorbés les pigments polaires, pour séparer les pigments polaires du lit fixe de cette troisième colonne, et recueillir la solution d'alimentation déchargée de la troisième colonne et l'évaporer à sec, de manière à récupérer les pigments polaires,
- et envoyer un solvant de régénération (méthanol, éthanol, acétone ou acétate d'éthyle) dans une quatrième colonne où est adsorbé l'acide bétulinique, pour séparer l'acide bétulinique du lit fixe de cette quatrième colonne, et recueillir la solution d'alimentation déchargée de la quatrième colonne et l'évaporer à sec, de manière à récupérer l'acide bétulinique.

8. Appareillage permettant de produire simultanément de la bétuline ultra-pure et de l'acide bétulinique de haute pureté à partir d'écorce de bouleau, lequel appareillage comprend :
- une première et une troisième colonne (I-A et I-B), pour l'adsorption et la séparation des pigments polaires contenus dans une solution d'alimentation ;
- une vanne principale (V21) pour envoyer la solution d'alimentation vers une conduite de charge (11) de la première colonne ou vers une conduite de charge (31) de la troisième colonne ;
- une deuxième colonne (II-A), raccordée à une conduite de décharge (12) de la première colonne, pour l'adsorption de l'acide bétulinique et la séparation de celui-ci de la solution d'alimentation déchargée de la première colonne ;
- une quatrième colonne, raccordée à une conduite de décharge (32) de la troisième colonne, pour l'adsorption de l'acide bétulinique et la séparation de celui-ci de la solution d'alimentation déchargée de la troisième colonne ;
- une conduite (20) de décharge de bétuline, raccordée aux conduites de décharge (13) et (33) des deuxième et quatrième colonnes, qui est munie d'une vanne (V25) de commutation de décharge, et qui sert à décharger la solution d'alimentation qui contient de la bétuline et d'avec laquelle les pigments polaires et l'acide bétulinique ont été séparés dans les première, deuxième, troisième et quatrième colonnes (I-A, I-B, II-A et II-B) ;
- une conduite (40) d'alimentation en solvant de régénération, raccordée aux conduites de décharge des première et troisième colonnes, qui est munie d'une deuxième vanne de commutation (V23) et qui permet d'envoyer un premier solvant de régénération (I), au moyen de cette deuxième vanne de commutation (V23), dans la première colonne (I-A) ou dans la troisième colonne (I-B) ;
- une conduite (50) de décharge de pigments polaires, raccordée aux conduites de charge (11) et (31) des première et troisième colonnes, qui est munie d'une première vanne de commutation (V22) et qui permet de décharger le premier solvant de régénération (I) contenant les pigments polaires, qui se trouve dans les première et troisième colonnes (I-A) et (I-B), au moyen de cette première vanne de commutation (V22) ;
- une conduite (60) d'alimentation en solvant de régénération, raccordée aux conduites de décharge (13) et (33) des deuxième et quatrième colonnes, qui est munie d'une quatrième vanne de commutation (V26) et qui permet d'envoyer un deuxième solvant de régénération (II), au moyen de cette quatrième vanne de commutation (V26), dans la deuxième colonne (II-A) ou dans la quatrième colonne (II-B) ;
- et une conduite (70) de décharge d'acide bétulinique, raccordée aux conduites de décharge (12) et (32) des première et troisième colonnes, qui est munie d'une troisième vanne de commutation (V24) et qui permet de décharger le deuxième solvant de régénération (II) contenant l'acide bétulinique, qui se trouve dans les deuxième et quatrième colonnes (II-A) et (II-B), au moyen de cette troisième vanne de commutation (V24).
